# EUROPÄISCHE PATENTSCHRIFT

(11) **EP 1 656 077 B1**
(45) Veröffentlichungstag und Bekanntmachung des Hinweises auf die Patenterteilung: **30.03.2011**
(21) Anmeldenummer: 04764304.4
(22) Anmeldetag: 19.08.2004
(51) Int. Cl.: A61C 5/04

(54) **GUTTAPERCHASPITZE**
GUTTA PERCHA TIP
POINTE A BASE DE GUTTA-PERCHA

(30) Priorität: 19.08.2003 DE 10338440
(43) Veröffentlichungstag der Anmeldung: 17.05.2006
(60) Teilanmeldung: 10010405.8
(73) Patentinhaber: Mannschedel, Werner, 89129 Langenau (DE); Müller, Barbara, 89129 Langenau (DE)
(72) Erfinder: Mannschedel, Werner, 89129 Langenau (DE); Müller, Barbara, 89129 Langenau (DE)
(74) Vertreter: Forstmeyer, Dietmar
(86) Internationale Anmeldenummer: PCT/EP2004/009320
(87) Internationale Veröffentlichungsnummer: WO 2005/018478

(56) Entgegenhaltungen:
- WO-A-2004/060192
- FR-A- 2 613 930
- US-A- 1 757 595
- US-A- 3 899 830

## Beschreibung

Die vorliegende Erfindung liegt auf dem Gebiet der Hilfsmittel für die Zahnheilkunde und betrifft eine Guttaperchaspitze zur Füllung eines Zahnwurzelkanalsystems.

Bei der Wurzelkanalbehandlung ist ein möglichst hermetischer Verschluss mit einem das Gewebe nicht irritierenden Füllmaterial angestrebt. Ein ideales Wurzelkanalfüllmittel sollte das periapikale Gewebe nicht reizen, den Wurzelkanal lateral und vertikal dicht verschließen, volumenbeständig sein und im Wurzelkanal nicht schrumpfen. Ferner sollte es das Bakterienwachstum nicht begünstigen, möglichst bakteriostatisch sein, dabei aber biologisch kompatibel und nicht toxisch sein.

Es hat sich gezeigt, dass die Wurzelkanalfüllung mit Guttaperchastiften und einem Dichtmittel ("Sealer") die biologisch günstigste und langfristig die sicherste Methode ist. Ein Guttaperchastift zeichnet sich durch die thermoplastische Verformbarkeit des Materials aus, die eine gute Verarbeitbarkeit unter leichter Erwärmung, z.B. bei Körpertemperatur d.h. 37°C, erlaubt und dadurch eine besonders sichere Füllung eines Wurzelkanals ermöglicht. Üblicherweise hat eine derartige Guttaperchaspitze einen Guttaperchagehalt von etwa 20 Gew.-% + 10 Gew.-%. Damit unterscheidet sich ein Guttaperchastift bzw. eine Guttaperchaspitze von einem Retentionsstift oder Post, der aus einem festen Material, wie z.B. einem Metall oder einer Metallegierung, besteht und zur Befestigung von Zahnprothesen, wie z.B. Kronen oder Brücken, in einer Zahnkavität, wie z.B. in den oberen 2/3 eines Wurzelkanals, verankert wird. Weit verbreitete Methoden zum Einbringen von Guttapercha in den Wurzelkanal sind die laterale Kondensation, die vertikale Kondensation, die thermomechanische Kondensation, sowie Injektionstechniken.

Bei dem oft angewandten Verfahren der lateralen Kondensation beginnt die Wurzelkanalfüllung mit der Auswahl eines Guttaperchahauptstiftes ("Masterpoint"), welcher zunächst in den Wurzelkanal eingeführt wird. Anschließend wird ein zweiter Guttaperchastift in den Wurzelkanal eingeführt und mittels eines hakenförmigen Werkzeugs, dem sog. Spreader, verformt und mit dem Masterpoint kondensiert. Dieser Vorgang wird solange wiederholt bis eine homogene wurzelkanalfüllung erreicht ist. Koronal überstehende Reste der Guttaperchastifte werden anschließend abgetrennt. Aufgrund der schlechten Dichteigenschaffen von Guttapercha ist für eine hermetische Abdichtung des Wurzelkanals die Anwendung eines zusätzlichen Dichtmittels unerlässlich, welches beispielsweise auf die Oberfläche der Guttaperchaspitzen aufgebracht wird.

In WO 2004/060192 A wird ein weiteres Verfahren beschrieben, bei dem ein einzelner Guttaperchastift verwendet wird, der einen konischen Bereich aufweist, der angepasst ist, um den Wurzelkanal auszufüllen, und netzförmig gemustert ist, um die Fläche seiner Oberfläche zu vergrößern.

Es hat sich gezeigt, dass es insbesondere bei dem oben gezeigten Verfahren der lateralen Kondensation vorkommen kann, dass eine durch den Spreader bewirkte Verformung der Guttaperchaspitzen oftmals nicht in befriedigender Weise erfolgen kann, weil die Guttaperchaspitzen, welche im Querschnitt rund sind, einer verformung durch den manuell geführten Spreader ausweichen. Hierdurch kann eine befriedigende laterale Kondensation und homogene Wurzelkanalfüllung nicht immer sichergestellt werden. Zumindest ist ein einwandfreies Ergebnis der Wurzelkanalfüllung nur mit vergleichsweise hohem Zeitaufwand zu verwirklichen und setzt nicht zuletzt eine entsprechende Geschicklichkeit des Anwenders voraus.

Bei den herkömmlichen verfahren zum Füllen des Wurzelkanalaystems mit Guttaperchaspitzen, insbesondere der lateralen Kon-densation, ist es zur Vermeidung einer Reizung des periapikalen Gewebes durch Füllmittel oftmals angezeigt, einen sog. apikalen Stop zu präparieren, d. h. der üblichen konischen Kanalkonfiguration ein definiertes Ende zu setzen. Eine vollständige Füllung des Wurzelkanals mit Guttapercha ist aufgrund des geringen zur Verfügung stehenden Raums oftmals nur schwierig zu bewerkstelligen.

Ferner können bei den herkömmlichen Verfahren zum Einbringen von Guttapercha in den Wurzelkanal aufgrund von eingeschlossener Luft oder überschüssigem Dichtmittel erhebliche Probleme entstehen, die dazu führen können, dass die Wurzelkanalbehandlung nicht zu dem gewünschten Erfolg führt. Auch ist es beim Einsatz von herkömmlichen Guttaperchastiften nicht gewährleistet, daß genügend Sealer in die Dentinkanälchen eingebracht wird, um einen vollständigen Verschluß zu gewährleisten und damit ein Eindringen von Flüssigkeiten und Keimen zu verhindern.

Demgegenüber besteht die Aufgabe der vorliegenden Erfindung darin, Guttaperchaspitzen zur Verfügung zu stellen, mit denen die oben genannten Probleme beseitigt bzw. vermindert werden können.

Guttaperchastifte sind an sich bekannt und können in einer Vielzahl von Ausführungsformen im Handel bezogen werden. In der vorliegenden Erfindung bedeutet der Begriff "Guttaperchaspitze" bzw. "Guttaperchastift" insbesondere eine Spitze oder einen Stift zur Füllung eines Zahnwurzelkanalsystems, welche Guttapercha umfaßt, wobei Guttapercha ein bekannter, aus Baumharzen gewonnener Naturstoff auf Polyisoprenbasis ist. Neben Guttapercha kann die Guttaperchaspitze bzw. der Guttaperchastift (im weiteren Guttaperchaspitze genannt) übliche Zusätze enthalten, zum Beispiel anorganische substanzen, wie z.B, anorganische Füllstoffe wie Zinkoxid, Röntgenkontrastmittel wie Schwermetallaalze, insbesondere Hariumaulfat, oder organische Substanzen, wie z.B. Wachse. Üblicherweise enthält eine Guttaperchaspitze etwa 70 Gew.-% ZnO, etwa 10 Gew.-% BaSO₄ und etwa 1-4 Gew.-% Wachse.

Gemäß einer ersten Ausführungsform der Erfindung wird eine Guttaperchaspitze zur Füllung eines Zahnwurzelkanalsystems bereitgestellt, welche dadurch gekennzeichnet ist, dass an der Oberfläche der Guttaperchaspitze wenigstens eine Abragung ausgebildet ist, wobei die Abragung in Form eines Widerhakens, eines Noppens oder Lappens oder eines umlaufenden Ringes ausgebildet, ist order die Abragung in form einer Schraubenwindung ausgebildet ist. Dadurch kann z.B. ein Wegrollen der Guttaperchaspitzen bei der Füllung des Zahnwurzelkanalsystems verhindert werden. Da sich die Abragungen bei Kontakt mit der Wurzelkanalwand verbiegen und einen flächigen Kontakt mit dieser erstellen, wird so eine größere Kontaktoberfläche bereitgestellt, die eine versehentliche Bewegung oder gar Entfernung der Guttaperchaspitze aus dem Wurzelkanal heraus verhindert. Übliche Guttaperchaspitzen haben den Nachteil, daß sie einen schlechten Kontakt mit dem Wurzelkanal und dem Sealer eingehen. So kann es bei üblichen Guttaperchaspitzen vorkommen, daß durch Manipulationen am koronalen Ende einer in einen Wurzelkanal eingesetzten Guttaperchaspitze, wie z.B. zum Verkürzen der Spitze oder Füllung zum Einsetzen eines Posts, die Guttaperchaspitze im Wurzelkanal bewegt bzw, daraus entfernt wird. Eine Abragung verbessert die Verbindung zwischen Guttaperchaspitze und Sealer bzw. Guttaperchaspitze und Dentin in vorteilhafter Weise. Durch eine vorgegebene Stärke der Abragung kann außerdem eine gewünschte Stärke der Sealerschicht eingestellt werden. Auch kann durch die Abragungen ein Sealer weiter in den Wurzelkanal hinein transportiert und im Wurzelkanal verdichtet werden, was nicht nur zu einer vorteilhaften Ausfüllung des Wurzelkanals führt, sondern auch eine Verdrängung des Sealers in Dentinkanäle bewirkt, wodurch diese insbesondere gegenüber Flüssigkeiten und Keimen vorteilhaft verschlossen werden. Weiterhin wird ein Verkeilen, Verspreizen bzw. Verklemmen mehrerer Guttaperchaspitzen im Wurzelkanal durch mindestens eine auf der Oberfläche jeder einzelnen Spitze ausgebildeten Abragung erleichtert, was ebenfalls die Fixierung der Spitzen im Wurzelkanal vorteilhaft verbessert.

Weiterhin vorteilhaft ist eine solche Abragung in Form einer Schraubenwindung ausgebildet. Insbesondere wird dadurch eine Fixierung des Guttaperchastiftes im Wurzelkanal sichergestellt, weil die Schraubenwindungen verbogen werden und eine großflächige Kontaktfläche mit der Wurzelkanalwand erzeugen.

Bevorzugt sind gleichzeitig mehrere gleiche oder verschiedene Abragungen auf der Oberfläche einer erfindungsgemäßen Guttaperchaspitze vorgesehen. Mehrere Abragungen auf der Oberfläche einer Guttaperchaspitze können auch verschiedene Größen oder Stärken aufweisen. Bevorzugt ändert sich die relative Größe oder Stärke mehrerer Abragungen auf der Oberfläche einer Guttaperchaspitze entlang ihrer Längsachse, d.h. in Richtung des apikalen bzw. koronalen Endes der Guttaperchaspitze. So kann eine Guttaperchaspitze z.B. im apikalen Bereich einen relativ dünneren umlaufenden Ring, in einem mittleren Bereich einen stärkeren Ring und im koronalen Bereich einen noch stärkeren Ring aufweisen.

Bei einer zweiten vorteilhaften Ausführungsform der Guttaperchaspitze der vorliegenden Erfindung weist sie wenigstens einen Abschnitt mit einer Querschnittsfläche mit einem nicht-runden (nicht-kreisförmigen) Umfang auf. Mit anderen Worten, die Guttaperchaspitze weist wenigstens einen Abschnitt mit einem nicht-runden Querschnitt auf, durch welchen die Rolleigenschaft der Guttaperchaspitze beseitigt oder zumindest vermindert ist. Der Ausdruck "Querschnittsfläche", wie er hier verwendet wird, beschreibt vorzugsweise einen Schnitt durch die Guttaperchaspitze, welcher senkrecht zur Spitzenrichtung verläuft. Gemäß einer bevorzugten Ausführungsform weist die Guttaperchaspitze eine eckige Form auf.

Bei einer besonders vorteilhaften Ausgestaltung der zweiten Ausführungsform der Guttaperchaspitze der Erfindung kann die Guttaperchaspitze in Spitzenrichtung mehrere Abschnitte umfassen, wobei wenigstens ein Abschnitt eine Querschnittsfläche mit einer Umfangsform aufweist, welche von der Umfangsform der Querschnittsfläche eines anderen Abschnitts verschieden ist. So kann die Guttaperchaspitze beispielsweise apikal Querschnittsflächen mit einem kreisförmigen Umfang aufweisen. Dieses kann in der dreidimensionalen Betrachtung einer konischen Form der Guttaperchaspitze in diesem Bereich entsprechen. Ferner kann die Guttaperchaspitze koronal Querschnittsflächen mit einem nicht-runden, beispielsweise eckigen, Umfang aufweisen. Hierdurch kann in vorteilhafter Weise die Rolleigenschaft der Guttaperchaspitzen in den nicht-apikalen Abschnitten des Zahnwurzelkanalsystems mit größerem Hohlraumvolumen beseitigt oder zumindest vermindert werden. Der Wurzelkanal selbst hat in der Regel keinen runden Querschnitt, so daß eine Füllung mit einer Guttaperchaspitze mit nicht-rundem Querschnitt zu einer vorteilhaften Ausfüllung des Wurzelkanals führt.

Die Ausdrücke "apikal" und "koronal", wie sie hier verwendet werden, beziehen sich auf die Anordnung der Guttaperchaspitze im Zahnwurzelkanalsystem.

In einer weiteren beispielhaften Ausführungsform können Abschnitte mit Querschnittsflächen mit rundem (kreisförmigem) Umfang und nicht-rundem (nicht-kreisförmigem) Umfang alternierend angeordnet sein.

Erfindungsgemäß ist es bevorzugt, wenn der Umfang der Querschnittsfläche teilweise oder vollständig in Gestalt eines geschlossenen Polygonzugs, d. h. miteinander verbundenen geraden Linien, die an einer Knickstelle ineinander übergehen, vorliegt. Die Knickstellen zwischen den Geraden korrespondieren in der dreidimensionalen Form mit in Spitzenrichtung sich erstreckenden Kanten der Oberfläche der Guttaperchaspitze, und entsprechen den Ecken eines eckigen Umfanges der Querschnittfläche der Guttaperchaspitze. Eine derartige Ausführung der Guttaperchaspitzen ist wegen der Kanten besonders vorteilhaft zur Verhinderung des Wegrollens der Guttaperchaspitzen bei der Füllung des Zahnwurzelkanalsystems und erlaubt eine gute Fixierung einer Guttaperchaspitze im Wurzelkanal. Gleichzeitig haben die ebenen Flächen einer derartigen Ausführung der Guttaperchaspitze den Vorteil, daß mehrere Spitzen besonders gut gegeneinander mit großer Auflagefläche fixiert werden können, z.B. bei lateraler Kondensation. Weiterhin erlauben die ebenen Flächen eine gute Raumausfüllung des Wurzelkanals durch die Guttaperchaspitzen, da zwei Guttaperchaspitzen über eine gemeinsame coplanare gerade Fläche dicht, d.h. ohne Zwischenraum, miteinander verbunden werden können. Auch können derartige Guttaperchaspitzen besonders gut verkeilt, verspreizt bzw. verklemmt werden. Ein weiterer Vorteil großer ebener Oberflächen ist, daß sie aufgrund größerer Reibung nicht so leicht an anderen Oberflächen vorbeigleiten wie es kleinere Oberflächen tun. So wird ein aneinander Vorbeigleiten von Guttaperchaspitzen erschwert, was ein ungewolltes Verrutschen von schon plazierten Spitzen verhindert. Weiterhin haben große Oberflächen den Vorteil, daß sie auch ohne Kondensation gut schließen, was im Hinblick auf eine gute Abdichtung des Wurzelkanals vorteilhaft ist.

Gemäß einer dritten Ausführungsform der Erfindung, welche insbesondere mit der ersten und/oder zweiten Ausführungsform der Erfindung kombiniert werden kann,weist die Oberfläche der Guttaperchaspitze in verschiedenen Abschnitten eine unterschiedliche Rauhigkeit zur Verminderung der Rolleigenschaften einer Guttaperchaspitze auf. Hierdurch kann die Guttaperchaspitze in vorteilhafter Weise beispielsweise im apikalen Bereich des Zahnwurzelkanalsystems eine geringere Rauhigkeit aufweisen als im nicht-apikalen Bereich des Zahnwurzelkanalsystems. Zur Erhöhung der Rauhigkeit der Oberfläche der Guttaperchaspitze kann.auf der Oberfläche der Guttaperchaspitze, wenigstens eine, insbesondere musterförmige, dreidimensionale Struktur ausgebildet sein. Musterförmige dreidimensionale Strukturen sind durch regelmäßig wiederholte Strukturelemente bestimmt, die zum Beispiel in einer Form von Vertiefungen oder Ausbuchtungen vorliegen, wohingegen bei unregelmäßigen dreidimensionalen Strukturen die Strukturelemente zufällig angeordnet sind. Eine solche dreidimensionale Struktur zur Erhöhung der Rauhigkeit der Oberfläche der Guttaperchaspitze liegt vorzugsweise in Form einer Bienenwabenstruktur vor. Hierdurch kann nicht nur ein Wegrollen der Guttaperchaspitze in effektiver Weise verhindert werden, sondern zudem kann auch die Oberfläche der Guttaperchaspitze vergrößert werden, so dass die Kondensation der Guttaperchaspitzen begünstigt ist. Weiterhin verbessert eine solche dreidimensionale Struktur zur Erhöhung der Rauhigkeit der Oberfläche die Fixierung einer Guttaperchaspitze im Wurzelkanal, wodurch insbesondere der Masterpoint apikal fixiert werden kann, um einen vorteilhaften Verschluß des Wurzelkanals am Apex sicherzustellen. Weitere Vorteile ergeben sich beim Verkeilen, verspreizen, Verklemmen und/oder Verzahnen, wodurch Guttaperchaspitzen nicht nur im Wurzelkanal, sondern auch gegeneinander fixiert werden. Auch wird das aneinander Vorbeigleiten von mehreren Guttaperchaspitzen bzw. das Verrutschen einer Guttaperchaspitze im Wurzelkanal durch eine Oberflächenstruktur vorteilhaft erschwert. Ebenfalls wird die Verbindung zwischen Guttaperchaspitze und Sealer durch eine dreidimensionale Oberflächenstruktur vorteilhaft verbessert.

Gemäß einer vierten Ausführungsform der Erfindung, welche insbesondere mit der ersten, zweiten und/oder dritten Ausführungsform der Erfindung kombiniert werden kann, ist die Guttaperchaspitze in einer Richtung z.B. parallel zur Spitzenrichtung mit wenigstens einem Durchgangskanal versehen. Ein derartiger Durchgangskanal kann z.B. vollständig innerhalb der Guttaperchaspitze verlaufen; alternativ hierzu kann der Durchgangskanal auch in Form einer rinnenförmigen Aussparung der Oberfläche der Füllspitze ausgebildet sein. Durch derartige Durchgangskanäle können in vorteilhafter Weise Luft und/oder Fluide, insbesondere der Sealer, aus dem Wurzelkanalsystem entweichen, so dass mögliche Komplikationen aufgrund etwaig verbliebener Luft im Wurzelkanalsystem bzw. wegen überschüssigem Sealer vermieden werden können. Dies trägt wesentlich zu einem positiven Behandlungsergebnis bei.

Ferner ist es dadurch möglich, eine Menge an Sealer einzusetzen, die gewährleistet, daß die Dentinkanälchen vollständig und dauerhaft verschlossen werden. Weiterhin wird so eine Überfüllung mit Sealer vermieden, so daß insbesondere ein Austreten des Sealers aus dem Apex verhindert werden kann.

Gemäß einer fünften Ausführungsform der Erfindung, welcher insbesondere mit der ersten, zweiten, dritten und/oder vierten Ausführungsform der Erfindung kombiniert werden kann, weist die Guttaperchaspitze an ihrer Oberfläche wenigstens eine, insbesondere parallel zur Spitzenrichtung verlaufende, Struktur wie eine Versteifungsrippe zur Versteifung der Guttaperchaspitze auf. Bevorzugt wird die Struktur in Form einer parallel zur Spitzenrichtung verlaufenden Versteifungsrippe bereitgestellt. Mittels derartiger Strukturen kann das Einbringen einer Guttaperchaspitze in das Zahnwurzelkanalsystem deutlich erleichtert werden. Weiterhin verbessert eine derartige Struktur sowohl die Steifigkeit als auch die Festigkeit der Guttaperchaspitze vorteilhaft, während die plastische Formbarkeit der Guttaperchaspitze erhalten bleibt. Alternativ kann die Festigkeit der Guttaperchaspitze auch durch Einarbeiten eines üblichen Versteifungsmaterials, wie z.B. von verstärkenden Fasern aus Glasfaser oder Kohlenstoff sowie Polymeren, erhöht werden.

Die erfindungsgemäße Guttaperchaspitze kann in vorteilhafter Weise durch Gießen, insbesondere Spritzgießen, von fluidem Guttapercha in einer Gussform zur Herstellung eines Guttapercha-Gussprodukts, und anschließendem Verfestigen des Guttapercha-Gussprodukts zur Herstellung der Guttaperchaspitze hergestellt werden. Hierbei ist es besonders bevorzugt, wenn das fluide Guttapercha in einer Gussform gegossen, welche ein Negativabdruck eines Positivabdrucks eines Zahnwurzelkanalsystems ist. Mit anderen Worten, zunächst wird das Zahnwurzelkanalsystem, beispielsweise durch ein Einbringen einer Silikonmasse, abgeformt um den Positivabdruck zu erstellen. Anschließend wird von diesem Positivabdruck ein Negativabdruck erzeugt, d. h. eine Hohlform, welche dem Hohlraum des Zahnwurzelkanalsystems entspricht. Dann wird der Positivabdruck mit fluidem Guttapercha ausgegossen und zur Herstellung der Guttaperchaspitze verfestigt. Die Guttaperchaspitze weist als eine zum Hohlraum des Zahnwurzelkanalsystems komplementäre Form auf.

Alternativ kann die Guttaperchaspitze durch eine spanabhebende Bearbeitung eines festen Guttapercha-Rohlings hergestellt werden. Beispielsweise wird die Guttaperchaspitze hierbei aus einem Guttapercha-Rohling gefräst. In besonders vorteilhafter Weise wurde hierbei der Hohlraum des Zahnwurzelkanalsystems zuvor in geeigneter Weise räumlich vermessen, und die Guttaperchaspitze wird anhand dieser Messdaten aus dem Guttapercha-Rohling gefräst, so dass - ähnlich dem Gießen der Guttaperchaspitze mittels eines Negativabdrucks - eine Guttaperchaspitze mit einer zum Hohlraum des Zahnwurzelkanalsystems komplementären Form hergestellt wird.

Weiterhin kann die Guttaperchaspitze durch eine nichtspanabhebende Bearbeitung eines festen Guttapercha-Rohlings hergestellt werden. Beispielsweise wird ein Guttapercha-Rohling mit einem geeigneten Presswerkzeug zur Formgebung der Guttaperchaspitzen verpresst. Diese Vorgehensweise bietet in besonders vorteilhafter Weise die Möglichkeit, Guttaperchaspitzen unter Verwendung verschiedener Ausgangsmaterialien herzustellen. So können beispielsweise Guttaperchaspitzen mit einer Oberfläche aus Guttapercha und einem Kern aus einem von Guttapercha verschiedenen Material, insbesondere flexible Materialien, wie Acrylate, Silikone, Polyethylene, Polypropylene, Metalle, Polyurethane, thermoplastische Elastomere, kautschukähnliche Verbindungen usw., hergestellt werden. In besonders vorteilhafter Weise wurde hierbei der Hohlraum des Zahnwurzelkanalsystems zuvor in geeigneter Weise räumlich vermessen, und die Guttaperchaspitze wird anhand dieser Messdaten aus dem Guttapercha-Rohling verpresst, so dass - ähnlich dem Gießen der Guttaperchaspitze mittels eines Negativabdrucks - eine Guttaperchaspitze mit einer zum Hohlraum des Zahnwurzelkanalsystems komplementären Form durch Verpressen hergestellt wird.

Überraschend wurde gefunden, daß eine Herstellung einer Guttaperchaspitze mit genau definierten dreidimensionalen Oberflächenstrukturen und/oder genau definierten Abragungen möglich ist, wenn eine solche Struktur und/oder Abragung entweder in der Gussform angelegt bzw. nachträglich aufgebracht wird.

### Kurze Beschreibung der Figuren

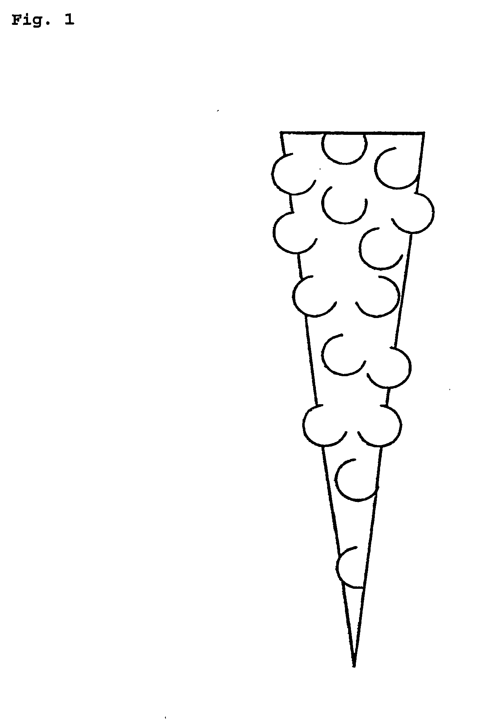

**Figur 1** zeigt eine bevorzugte Ausführungsform der erfindungsgemäßen Guttaperchaspitze, die mehrere Abragungen in Form von Noppen aufweist.

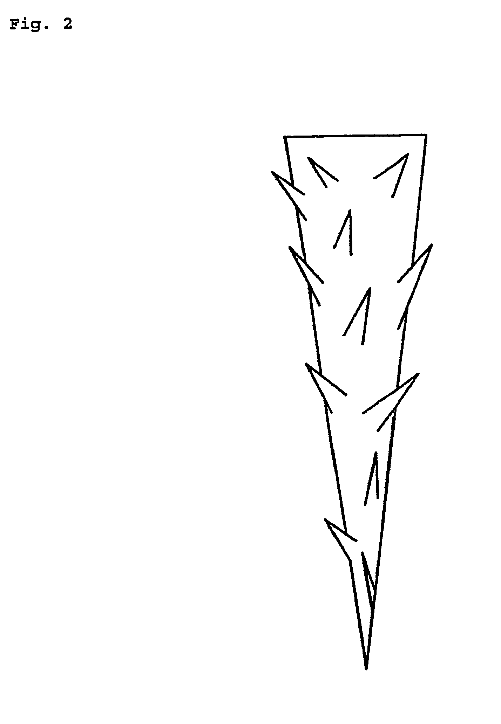

**Figur 2** zeigt eine bevorzugte Ausführungsform der erfindungsgemäßen Guttaperchaspitze, die mehrere Abragungen in Form von Widerhaken aufweist.

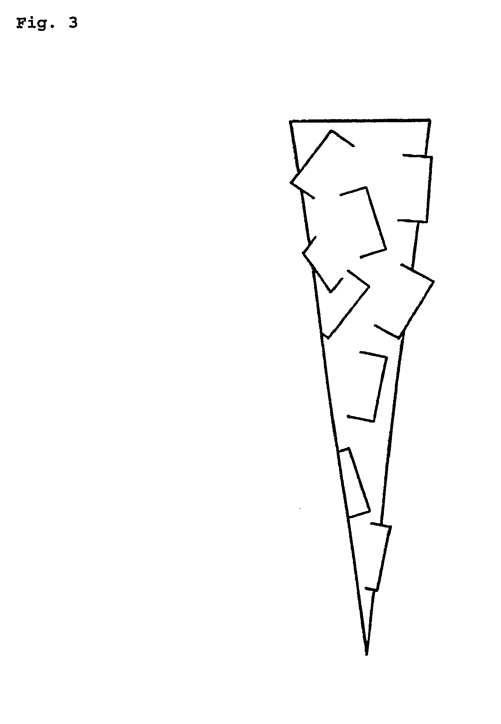

**Figur 3** zeigt eine bevorzugte Ausführungsform der erfindungsgemäßen Guttaperchaspitze, die mehrere Abragungen in Form von Lappen aufweist.

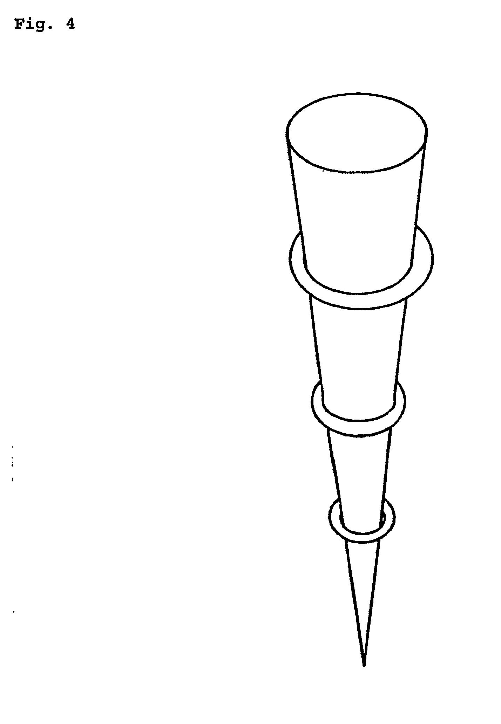

**Figur 4** zeigt eine bevorzugte Ausführungsform der erfindungsgemäßen Guttaperchaspitze, die mehrere Abragungen in Form von umlaufenden Ringen mit in apikaler Richtung abnehmenden Durchmessern aufweist.

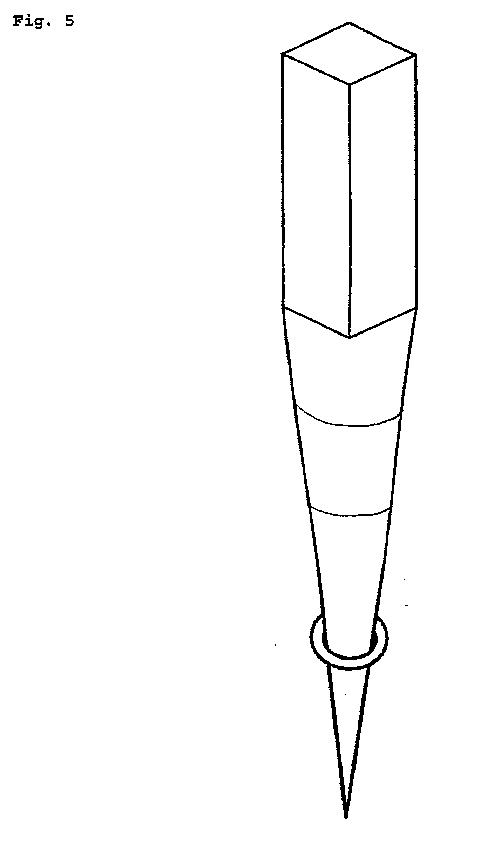

**Figur 5** zeigt eine bevorzugte Ausführungsform der erfindungsgemäßen Guttaperchaspitze, die eine Abragung in Form eines umlaufenden Ringes sowie einen Abschnitt mit einer Querschnittsfläche mit einem runden Umfang und einen Abschnitt mit einer Querschnittsfläche mit einem Umfang in Gestalt eines geschlossenen Polygonzugs aufweist.

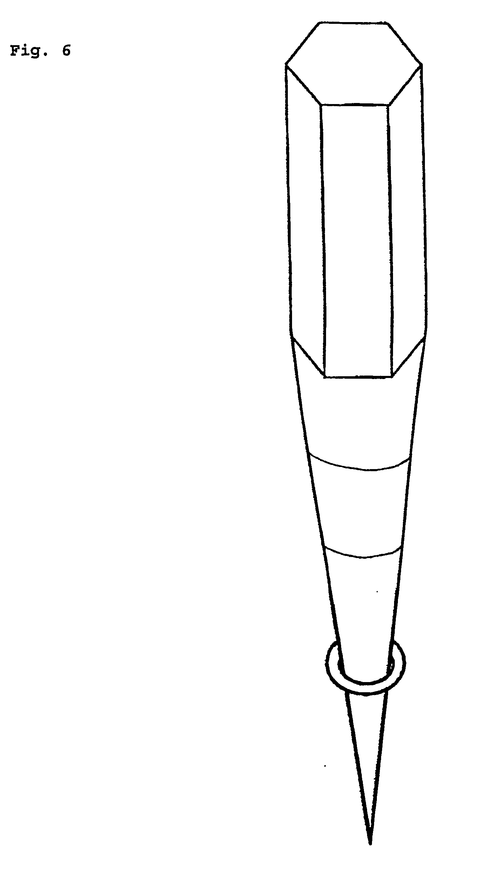

**Figur 6** zeigt eine weitere bevorzugte Ausführungsform der erfindungsgemäßen Guttaperchaspitze, die eine Abragung in Form eines umlaufenden Ringes sowie einen Abschnitt mit einer Querschnittsfläche mit einem runden Umfang und einen Abschnitt mit einer Querschnittsfläche mit einem Umfang in Gestalt eines geschlossenen Polygonzugs aufweist.

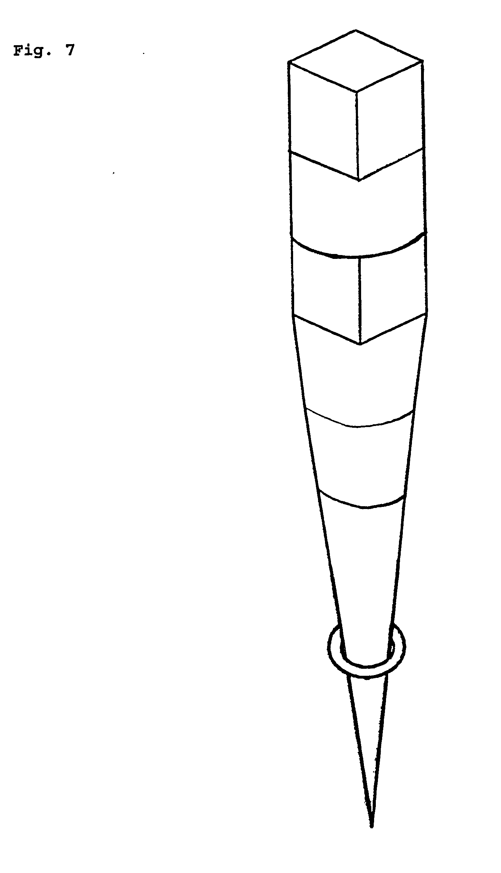

**Figur 7** zeigt eine bevorzugte Ausführungsform der erfindungsgemäßen Guttaperchaspitze, die eine Abragung in Form eines umlaufenden Ringes sowie zwei Abschnitte mit einer Querschnittsfläche mit einem runden Umfang und zwei Abschnitte mit einer Querschnittsfläche mit einem Umfang in Gestalt eines geschlossenen Polygonzugs aufweist.

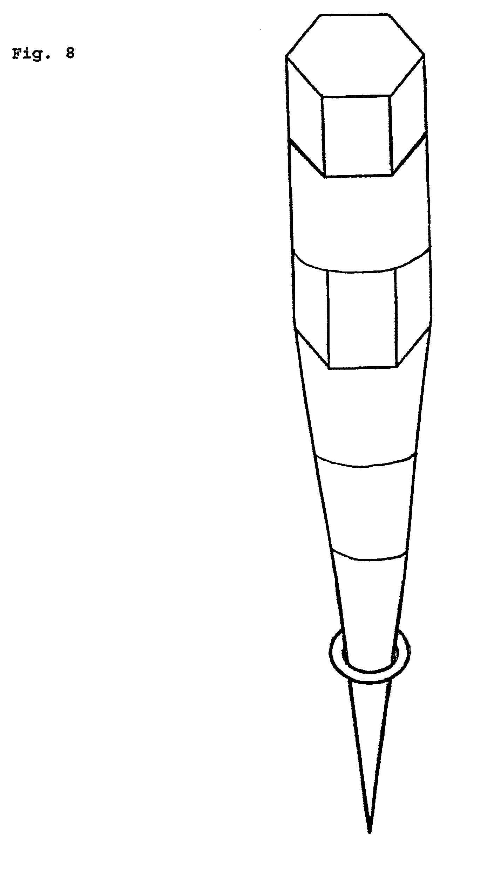

**Figur 8** zeigt eine weitere bevorzugte Ausführungsform der erfindungsgemäßen Guttaperchaspitze, die eine Abragung in Form eines umlaufenden Ringes sowie zwei Abschnitte mit einer Querschnittsfläche mit einem runden Umfang und zwei Abschnitte mit einer Querschnittsfläche mit einem Umfang in Gestalt eines geschlossenen Polygonzugs aufweist.

## Patentansprüche

1. Guttaperchaspitze zur Füllung eines Zahnwurzelkanalsystems, **dadurch gekennzeichnet, dass** an der Oberfläche der Guttaperchaspitze wenigstens eine Abragung ausgebildet ist, wobei die Abragung in Form eines Widerhakens, eines Noppens oder Lappens oder eines umlaufenden Ringes ausgebildet ist, order die Abragung in Form einer Schraubenwindung ausgebildet ist.

2. Guttaperchaspitze nach Anpruch 1, **dadurch gekennzeichnet, dass** die Guttaperchaspitze wenigstens einen Abschnitt mit einer Querschnittsfläche mit einem nicht-runden Umfang aufweist.

3. Guttaperchaspitze nach einem der Ansprüche 1 bis 2, **dadurch gekennzeichnet, dass** die Guttaperchaspitze in Spitzenrichtung mehrere Abschnitte umfasst, wobei wenigstens ein Abschnitt eine Querschnittsfläche mit einer Umfangsform, welche von der Umfangsform der Querschnittsfläche eines anderen Abschnitts verschieden ist, aufweist.

4. Guttaperchaspitze nach einem der vorhergehenden Ansprüche, **dadurch gekennzeichnet, dass** die Querschnittsfläche mit einem nicht-runden Umfang einen Umfang in Gestalt eines geschlossenen Polygonzugs hat.

5. Guttaperchaspitze nach einem der Ansprüche 1 bis 4, **dadurch gekennzeichnet, dass** die Oberfläche der Guttaperchaspitze in verschiedenen Abschnitten eine unterschiedliche Rauhigkeit aufweist.

6. Guttaperchaspitze nach Anspruch 5, **dadurch gekennzeichnet dass** die Oberfläche der Guttaperchaspitze wenigstens eine, insbesondere musterförmige, dreidimensionale Struktur aufweist.

7. Guttaperchaspitze nach Anspruch 6, **dadurch gekennzeichnet, dass** in verschiedenen Abschnitten der Guttaperchaspitze verschiedene dreidimensionale Strukturen ausgebildet sind.

8. Guttaperchaspitze nach Anspruch 6 oder 7, **dadurch gekennzeichnet, dass** die dreidimensionale Struktur eine Bienenwabenstruktur ist.

9. Guttaperchaspitze, nach einem der Ansprüche 1 bis 8, **dadurch gekennzeichnet, dass** sie in einer Richtung, insbesondere parallel zur Spitzenrichtung, mit wenigstens einem Kanal versehen ist.

10. Guttaperchaspitze nach Anspruch 9, **dadurch gekennzeichnet, dass** der Kanal in Form einer rinnenförmigen Aussparung der Oberfläche der Spitze ausgebildet ist.

11. Guttaperchaspitze, nach einem der Ansprüche 1 bis 10, **dadurch gekennzeichnet, dass** die Guttaperchaspitze an ihrer Oberfläche wenigstens eine, insbesondere parallel zur Spitzenrichtung verlaufende, Struktur zur Versteifung der Guttaperchaspitze aufweist.

12. Guttaperchaspitze nach einem der vorstehenden Ansprüche, **dadurch gekennzeichnet, dass** die Querschnittsfläche senkrecht zur Spitzenrichtung geschnitten ist.

## Claims

1. A gutta-percha point for filling a tooth root canal system, **characterised in that** at least one protrusion is formed on the surface of the gutta-percha point, wherein the protrusion is constructed in the form of a barb, a bump or lug or a circumferential ring, or the protrusion is constructed in the form of a helical winding.

2. A gutta-percha point according to claim 1, **characterised in that** the gutta-percha point has at least one portion having a cross-sectional area with a non-round circumference.

3. A gutta-percha point according to any one of claims 1 to 2, **characterised in that** the gutta-percha point has a plurality of portions, at least one portion having a cross-sectional area with a circumferential form that is different from the circumferential form of the cross-sectional area of a different portion.

4. A gutta-percha point according to any one of the preceding claims, **characterised in that** cross-sectional area with a non-round circumference has a circumference in the form of a closed polyline.

5. A gutta-percha point according to any one of claims 1 to 4, **characterised in that** the surface of the gutta-percha point has a different roughness in different portions.

6. A gutta-percha point according to claim 5, **characterised in that** the surface of the gutta-percha point has at least one, especially pattern-form, three-dimensional structure.

7. A gutta-percha point according to claim 6, **characterised in that** in different portions the gutta-percha point has different three-dimensional structures.

8. A gutta-percha point according to claim 6 or 7, **characterised in that** the three-dimensional structure is a honeycomb structure.

9. A gutta-percha point according to any one of claims 1 to 8, **characterised in that** it is provided with at least one channel in one direction, especially parallel to the direction of the point.

10. A gutta-percha point according to claim 9, **characterised in that** the channel is in the form of a channelled recess in the surface of the point.

11. A gutta-percha point according to any one of claims 1 to 10, **characterised in that** on its surface the gutta-percha point has at least one structure, running in particular parallel to the direction of the point, for reinforcing the gutta-percha point.

12. A gutta-percha point according to any one of the preceding claims, **characterised in that** the cross-sectional area is cut perpendicularly to the direction of the point.

## Revendications

1. Pointe de Gutta-Percha pour le remplissage d'un système de canal radiculaire, **caractérisée en ce qu'**au moins une saillie est réalisée sur la surface de la pointe de Gutta-Percha, la saillie ayant la forme d'un picot, d'une excroissance ou d'une languette ou d'une bague tournante ou la saillie ayant la forme d'une spire d'hélice.

2. Pointe de Gutta-Percha selon la revendication 1, **caractérisée en ce que** la pointe de Gutta-Percha présente au moins une portion ayant une aire de section transversale dont la périphérie est non circulaire.

3. Pointe de Gutta-Percha selon l'une des revendications 1 à 2, **caractérisée en ce que** la pointe de GuttaPercha comprend plusieurs portions dans la direction de la pointe, au moins une portion présentant une aire de section transversale ayant une forme périphérique différente de la forme périphérique de l'aire de section transversale d'une autre portion.

4. Pointe de Gutta-Percha selon l'une des revendications précédentes, **caractérisée en ce que** l'aire de section transversale ayant une périphérie non circulaire présente une périphérie sous forme de tracé polygonal fermé.

5. Pointe de Gutta-Percha selon l'une des revendications 1 à 4, **caractérisée en ce que** la surface de la pointe de Gutta-Percha présente, dans différentes portions, une rugosité différente.

6. Pointe de Gutta-Percha selon la revendication 5, **caractérisée en ce que** la surface de la pointe de GuttaPercha présente au moins une structure tridimensionnelle, en particulier en forme de motif.

7. Pointe de Gutta-Percha selon la revendication 6, **caractérisée en ce que**, dans différentes portions de la pointe de Gutta-Percha, sont formées des structures tridimensionnelles différentes.

8. Pointe de Gutta-Percha selon la revendication 6 ou 7, **caractérisée en ce que** la structure tridimensionnelle est une structure en nid d'abeilles.

9. Pointe de Gutta-Percha selon l'une des revendications 1 à 8, **caractérisée en ce qu'**elle est pourvue, dans une direction, en particulier parallèle à la direction de la pointe, d'au moins un canal.

10. Pointe de Gutta-Percha selon la revendication 9, **caractérisée en ce que** le canal est conçu sous la forme d'un évidement en forme de gouttière de la surface de la pointe.

11. Pointe de Gutta-Percha selon l'une des revendications 1 à 10, **caractérisée en ce que** la pointe de GuttaPercha présente sur sa surface au moins une structure, en particulier parallèle à la direction de la pointe, pour le renforcement de la pointe de Gutta-Percha.

12. Pointe de Gutta-Percha selon l'une des revendications précédentes, **caractérisée en ce que** l'aire de section transversale est coupée perpendiculairement à la direction de la pointe.
